# EUROPEAN PATENT APPLICATION

(11) **EP 4 510 047 A1**
(43) Date of publication of application: **19.02.2025**
(21) Application number: 22937392.3
(22) Date of filing: 12.04.2022
(51) Int. Cl.: G06N 20/00

(54) **MACHINE LEARNING PROGRAM, MACHINE LEARNING METHOD, AND INFORMATION PROCESSING DEVICE**

(71) Applicant: FUJITSU LIMITED, Kawasaki-shi, Kanagawa 211-8588 (JP)
(72) Inventor: FUKUTA, Shigeki, Kawasaki-shi, Kanagawa 211-8588 (JP); HIGUCHI, Hiroyuki, Kawasaki-shi, Kanagawa 211-8588 (JP); ASAI, Tatsuya, Kawasaki-shi, Kanagawa 211-8588 (JP); IWASHITA, Hiroaki, Kawasaki-shi, Kanagawa 211-8588 (JP)
(74) Representative: Hoffmann Eitle
(86) International application number: PCT/JP2022/017636
(87) International publication number: WO 2023/199413

(57) **Abstract**

An information processing apparatus extracts a feature related to a surface structure of a substance based on an atomic arrangement of the substance. The information processing apparatus trains a machine learning model that predicts information regarding a chemical reaction that occurs in a substance corresponding to an input explanatory variable using training data that includes, as explanatory variables, atomic arrangement information regarding the atomic arrangement of the substance and the extracted feature and includes, as an objective variable, information regarding the chemical reaction that occurs in the substance.

## Description

### TECHNICAL FIELD

The present invention relates to a machine learning program, a machine learning method, and an information processing apparatus.

### BACKGROUND ART

Physical properties of a chemical catalyst depend on a composition of a material and compatibility of chemical characteristics with a reactant, and it is difficult to analytically estimate the physical properties. In addition, there is a large number of selectable options such as a combination of catalyst materials, a mixing ratio, a surface structure, and the like, and it takes an enormous amount of time to actually carry out a chemical experiment or simulate a chemical reaction to perform a catalyst search for searching for a promising catalyst composition.

In recent years, with the development of artificial intelligence (AI), a method of estimating characteristics of a catalyst more easily than simulation of a chemical reaction has been employed to speed up the catalyst search.

### CITATION LIST

### PATENT DOCUMENT

Patent Document 1: Japanese Laid-open Patent Publication No. 2001-264309
Patent Document 2: Japanese Laid-open Patent Publication No. 05-288665
Patent Document 3: U.S. Patent Application Publication No. 2008/0168014
Patent Document 4: U.S. Patent Application Publication No. 2020/0340941

### NON-PATENT DOCUMENT

Non-Patent Document 1: FUJITSU LABORATORIES LIMITED, [online], searched on March 25, 2022, "Basic Concept of Wide Learning", "URL: https://widelearning.labs.fujitsu.com/ja/whatsWL/c001.html"

### SUMMARY OF INVENTION

### TECHNICAL PROBLEM

Meanwhile, since there are various possibilities of candidates for the catalyst search and a search range is enormous, it is difficult to automate the catalyst search. For example, since there are many types of search axes including elements that may form a catalyst, such as a catalyst material, a mixing ratio, a surface shape, and the like and the search exponentially increases, when the number of values that may be taken by each axis is X and the number or type of the search axes is n, the search range is Xⁿ, which is an enormous range.

In the AI for speeding up the catalyst search described above, "descriptors" representing chemical characteristics of catalysts, such as properties of catalysts and reactants and the like, are important as training data for training appropriate characteristics to estimate catalyst characteristics. In addition, appropriate features need to be trained to reduce the search range.

However, while candidates for the search axes are required to be prepared in a case of narrowing down the search axes by the AI, a large amount of effort is needed to manually extract the search axes, and the accuracy is not high due to the influence of omission of extraction, human error, preconceptions, and the like. As described above, it is difficult to speed up the catalyst search for searching for a promising catalyst composition.

In one aspect, an object is to provide a machine learning program, a machine learning method, and an information processing apparatus capable of searching for a promising catalyst composition at high speed.

### SOLUTION TO PROBLEM

According to a first idea, a machine learning program for causing a computer to execute a process includes extracting a feature related to a surface structure of a substance based on an atomic arrangement of the substance; and training a machine learning model that predicts information regarding a chemical reaction that occurs in a substance corresponding to an input explanatory variable using training data that includes, as explanatory variables, atomic arrangement information regarding the atomic arrangement of the substance and the extracted feature and includes, as an objective variable, information regarding the chemical reaction that occurs in the substance.

### EFFECTS OF INVENTION

According to one embodiment, a promising catalyst composition may be searched for at high speed.

### BRIEF DESCRIPTION OF DRAWINGS

FIG. 1 is a diagram for explaining an information processing apparatus according to a first embodiment.
FIG. 2 is a functional block diagram illustrating a functional configuration of the information processing apparatus according to the first embodiment.
FIG. 3 is a diagram for explaining a simulation data database (DB).
FIG. 4 is a diagram for explaining a structural characteristic data DB.
FIG. 5 is a diagram for explaining exemplary extraction of structural characteristic data.
FIG. 6 is a diagram for explaining combinations of descriptors.
FIG. 7 is a diagram for explaining generation of a machine learning model.
FIG. 8 is a diagram for explaining a causal relationship.
FIG. 9 is a diagram for explaining an exemplary output of the causal relationship.
FIG. 10 is a diagram for explaining exemplary prediction of the causal relationship.
FIG. 11 is a flowchart illustrating a process flow.
FIG. 12 is a diagram for explaining an exemplary hardware configuration.

### DESCRIPTION OF EMBODIMENTS

Hereinafter, embodiments of a machine learning program, a machine learning method, and an information processing apparatus according to the present invention will be described with reference to the drawings. Note that the present invention is not limited by the embodiments. In addition, the embodiments may be appropriately combined with each other unless otherwise contradicted.

### [First Embodiment]

### (Description of Information Processing Apparatus)

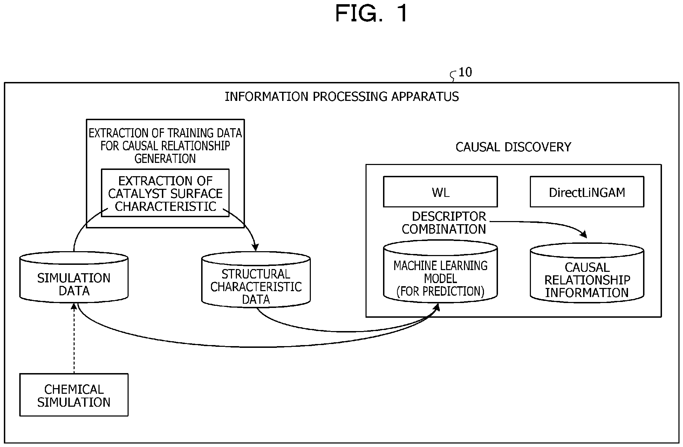

FIG. 1 is a diagram for explaining an information processing apparatus 10 according to a first embodiment. The information processing apparatus 10 illustrated in FIG. 1 is an exemplary computer device that efficiently executes a catalyst search for searching for a promising catalyst composition from combinations of catalyst materials having various options, such as a mixing ratio, a surface structure, and the like.

The information processing apparatus 10 extracts a feature related to a surface structure of a substance based on the atomic arrangement of the substance. The information processing apparatus 10 trains a machine learning model for predicting information regarding a chemical reaction that occurs in a substance corresponding to an input explanatory variable by using training data including atomic arrangement information regarding an atomic arrangement of a substance and an extracted feature as explanatory variables and including information regarding a chemical reaction that occurs in the substance as an objective variable. Here, the information processing apparatus 10 extracts a causal relationship between the objective variable and a descriptor used for an explanatory variable that largely affects the objective variable. As a result, the information processing apparatus 10 is enabled to efficiently execute the catalyst search to output a search result.

For example, as illustrated in FIG. 1, the information processing apparatus 10 obtains simulation data including coordinates of catalyst atoms, a physical property description of the catalyst atoms, and a physical property description of a reactant obtained by commonly used chemical simulation. The information processing apparatus 10 extracts, from the simulation data, structural characteristic data representing characteristics related to a surface structure of a catalyst.

Then, the information processing apparatus 10 generates a machine learning model by using the simulation data and the structural characteristic data as features and the information regarding a chemical reaction in analysis of a catalyst, such as "reaction energy", as an objective variable. For example, the information processing apparatus 10 extracts a combination of descriptors representing chemical characteristics of catalysts from the simulation data and the structural characteristic data. In addition, the information processing apparatus 10 generates causal relationship information including a causal relationship between the objective variable and the combination of individual descriptors used to train the machine learning model.

Thereafter, the information processing apparatus 10 executes chemical simulation on a prediction target catalyst, generates structural characteristic data, and extracts a descriptor for the prediction target catalyst. The information processing apparatus 10 extracts a characteristic that largely affects performance of the prediction target catalyst based on presence or absence of the combination of descriptors included in the causal relationship information. Note that as a result of the chemical simulation may be used from different systems or existing data.

As described above, the information processing apparatus 10 automatically extracts characteristics regarding a three-dimensional structure of a catalyst from the simulation data evaluated in various catalyst studies as a search area for catalyst researchers, exhaustively verifies combinations of features, and extracts a causal relationship for each condition group. Therefore, the information processing apparatus 10 is enabled to automatically extract a search axis, and to search for a promising catalyst composition at high speed. Furthermore, the searched promising catalyst composition may be applied to analysis of catalyst characteristics for finding a new catalyst and a reaction mechanism, and to an estimation system for estimating a catalyst composition.

### (Functional Configuration)

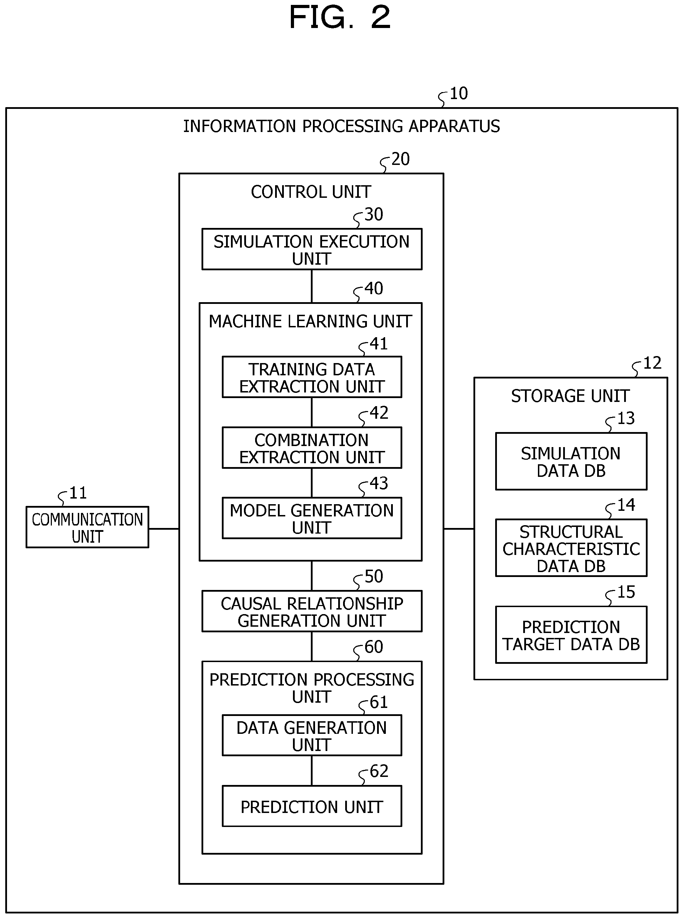

FIG. 2 is a functional block diagram illustrating a functional configuration of the information processing apparatus 10 according to the first embodiment. As illustrated in FIG. 2, the information processing apparatus 10 includes a communication unit 11, a storage unit 12, and a control unit 20.

The communication unit 11 is a processing unit that controls communication with another device, and is implemented by, for example, a communication interface. The communication unit 11 receives various instructions from an external device such as an administrator terminal, and transmits a prediction result generated by the control unit 20 to the external device such as the administrator terminal.

The storage unit 12 is a storage device that stores various types of data, programs to be executed by the control unit 20, and the like, and is implemented by, for example, a memory, a hard disk, or the like. The storage unit 12 includes a simulation data DB 13, a structural characteristic data DB 14, and a prediction target data DB 15.

The simulation data DB 13 is a database for storing atomic arrangement information of a substance, which is a result of chemical simulation. The data stored here may be data obtained from an external simulation terminal, or may be data generated by the control unit 20.

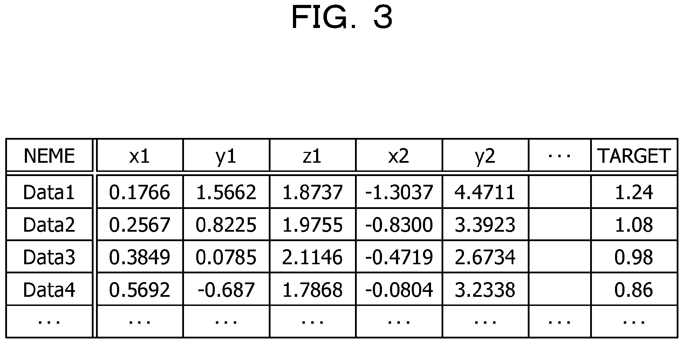

FIG. 3 is a diagram for explaining the simulation data DB 13. As illustrated in FIG. 3, the simulation data DB 13 stores, for each "name" indicating a catalyst to be simulated, coordinates of catalyst atoms, a physical property description of the catalyst atoms, a physical property description of a reactant, and the like. In the example of FIG. 3, descriptors such as "x1, y1, z1, ..., target" and the like are stored for "Data 1" indicating a catalyst. Here, "x1, y1, z1" indicates coordinates of an atom 1, and "target" indicates an objective variable specified in advance, such as reaction energy. Note that not only the coordinate data but also characteristic data of an atom at each lattice point, such as an atomic number, electronegativity, an ionic radius, and the like are included.

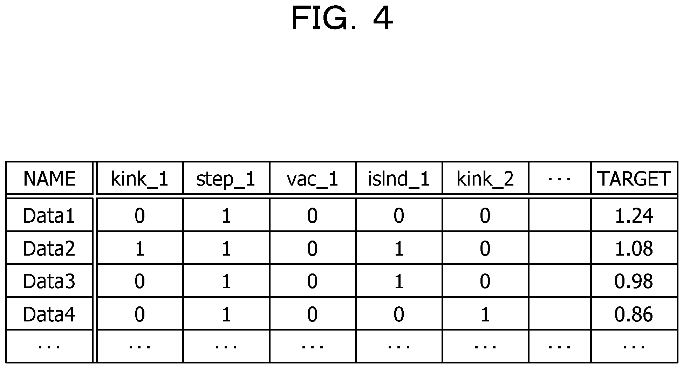

The structural characteristic data DB 14 is a database for storing structural characteristic data, which is generated by the control unit 20 and is related to structural characteristics of a catalyst. FIG. 4 is a diagram for explaining the structural characteristic data DB 14. As illustrated in FIG. 4, the structural characteristic data DB 14 stores feature data indicating a feature as a catalyst for each "name" indicating a catalyst.

In the example of FIG. 4, descriptors such as "kink_1, step_1, vac_1, island_1, ..., target" and the like are stored for "Data 1" indicating a catalyst. Here, "kink_1, step_1, vac_1, island_1" is data expressing, as a feature (1: Yes (applicable), 0: No (not applicable)), whether an atom or a space existing for each lattice position of a catalyst structure is kink, step, vacancy, or island. The "target" indicates an objective variable specified in advance, such as reaction energy.

The prediction target data DB 15 is a database for storing prediction target data related to a prediction target catalyst, which is data of a prediction target using a machine learning model. For example, the data to be stored in the prediction target data DB 15 may be data before being input to the chemical simulation, or may be data including a chemical simulation result and structural characteristic data.

The control unit 20 is a processing unit that takes overall control of the information processing apparatus 10, and is implemented by, for example, a processor or the like. The control unit 20 includes a simulation execution unit 30, a machine learning unit 40, a causal relationship generation unit 50, and a prediction processing unit 60. Note that the simulation execution unit 30, the machine learning unit 40, the causal relationship generation unit 50, and the prediction processing unit 60 are implemented by an electronic circuit such as a processor, a process executed by the processor, or the like.

The simulation execution unit 30 is a processing unit that executes the chemical simulation. For example, the simulation execution unit 30 executes atomic-level simulation, generates coordinates of catalyst atoms, a physical property description of the catalyst atoms, and a physical property description of a reactant, and stores them in the simulation data DB 13. Note that data related to a substance, gene information of a patient, material data to be subject to a chemical reaction, and the like may be adopted as simulation target data.

The machine learning unit 40 is a processing unit that includes a data extraction unit 41, a combination extraction unit 42, and a model generation unit 43, extracts a feature related to a surface structure of a substance based on the atomic arrangement of the substance, and trains a machine learning model using training data including atomic arrangement information of the substance and the feature.

The data extraction unit 41 is a processing unit that extracts feature data related to structural characteristics of a catalyst from the atomic arrangement information of the substance stored in the simulation data DB 13. Specifically, the data extraction unit 41 extracts surface structure information as a catalyst. For example, the data extraction unit 41 extracts surface structure information of a catalyst such as "Island" indicating an atom constituting an island of a certain scale, "Vacancy" indicating a lattice point to be a hole of a certain scale, "Step" indicating an atom that forms a step on the surface, "Kink" indicating an atom that hits a corner of the step, and the like, and stores the surface structure information in the structural characteristic data DB 14.

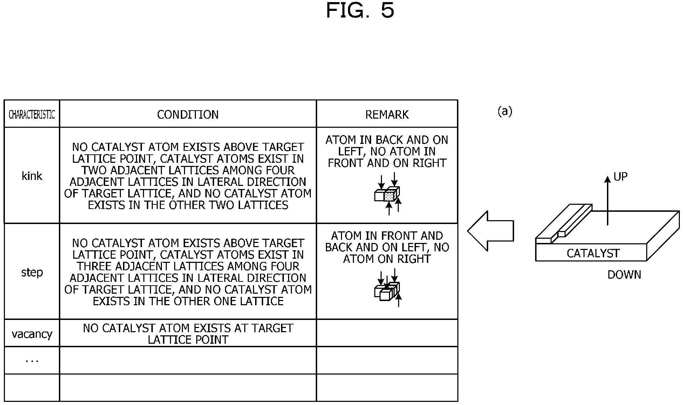

More specifically, the data extraction unit 41 defines a condition of the surface characteristics, and determines a state of each crystal lattice point, thereby extracting the surface structure information of the catalyst. FIG. 5 is a diagram for explaining exemplary extraction of the structural characteristic data. In FIG. 5, exemplary extraction in a case of adopting a parallelogram or cubic lattice structure, such as a simple lattice structure, will be described as an example.

As illustrated in FIG. 5, the data extraction unit 41 determines various conditions, such as "Kink", "Step", and the like, after defining that the side on which a catalyst crystal exists is directed downward and the surface is directed upward (see (a) in FIG. 5), and extracts structural characteristics. For example, the data extraction unit 41 determines "Kink" when no catalyst atom exists above the target lattice point, catalyst atoms exist in two adjacent lattices among four adjacent lattices in the lateral direction of the target lattice, and no catalyst atom exists in the other two lattices. That is, the data extraction unit 41 determines, as "Kink", an atom having atoms in the back and on the left and having no atoms in front and on the right.

Furthermore, the data extraction unit 41 determines "step" when no catalyst atom exists above the target lattice point, catalyst atoms exist in three adjacent lattices among four adjacent lattices in the lateral direction of the target lattice, and no catalyst atom exists in the other one lattice. That is, the data extraction unit 41 determines, as "step", an atom having atoms in front and back and on the left and having no atom on the right.

The combination extraction unit 42 is a processing unit that extracts a combination of descriptors using descriptors of the atomic arrangement information of the substance stored in the simulation data DB 13, the structural characteristic data stored in the structural characteristic data DB 14, and the like. Specifically, the combination extraction unit 42 extracts, as a hypothesis (knowledge chunk), a combination pattern of all descriptors (data items) from each descriptor of the atomic arrangement information and each descriptor of the structural characteristic data. Note that the descriptor may include information such as an atomic number and an atomic characteristic of an atom arranged at each lattice position, a direction and a force applied to the atom, an interaction between atoms, and the like.

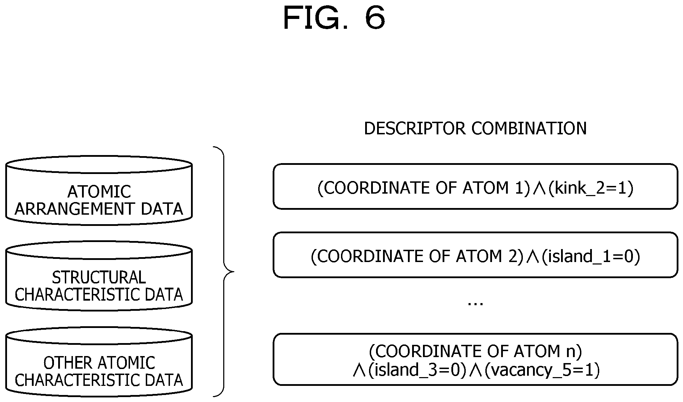

FIG. 6 is a diagram for explaining combinations of descriptors. As illustrated in FIG. 6, the combination extraction unit 42 extracts combinations such as "(coordinates of atom 1) and (kink_2 = 1)", "(coordinates of atom n) and (island_3 = 0) and (vacancy_5 = 1)", and the like using descriptors included in the atomic arrangement data obtained from simulation, the structural characteristic data, other atomic characteristic data input from the outside, and the like. The combinations of the descriptors are used not only as explanatory variables of a machine learning model but also for analysis of a causal relationship. The combination extraction unit 42 extracts explanatory variables of the machine learning model. The combination extraction unit 42 stores the extracted information in the storage unit 12, and outputs it to the causal relationship generation unit 50. Note that the method disclosed in, for example, Non-Patent Document 1 or the like may be adopted as a method of extracting combinations.

The model generation unit 43 is a processing unit that generates a machine learning model using the combinations of the descriptors generated by the combination extraction unit 42 and an objective variable specified in advance or an objective variable determined by a simulation result. The model generation unit 43 exhaustively checks the combinations of the descriptors, which are many factors of data to be analyzed, and automatically selects a combination deeply associated with the objective variable indicating an "energy amount for reaction" or the like, which is used for catalyst analysis, to generate a machine learning model (prediction model). Note that a prediction result may be described on the ground of the combinations of the factors.

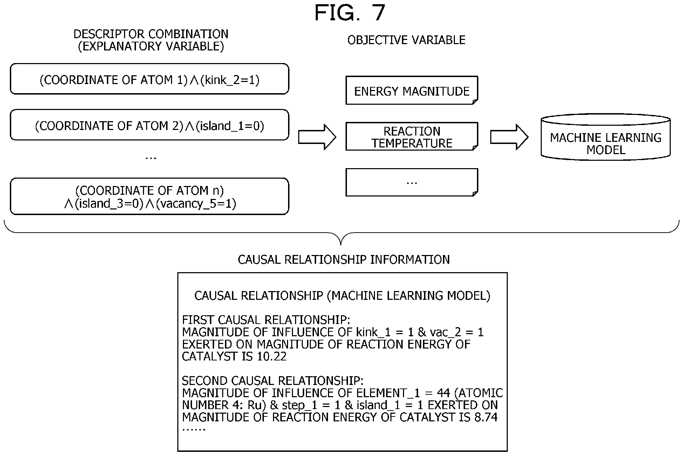

FIG. 7 is a diagram for explaining the generation of the machine learning model. As illustrated in FIG. 7, the model generation unit 43 sets, for each of the combinations of the descriptors generated by the combination extraction unit 42, for example, information regarding a chemical reaction in the catalyst analysis such as energy, a reaction rate, and the like as an objective variable (result), and generates a machine learning model. For example, the model generation unit 43 exhaustively searches for combinations of individual descriptors using the technique of Non-Patent Document 1, and extracts combinations that largely affect the objective variable (target). Then, the model generation unit 43 carries out machine learning of the machine learning model using the objective variable described above and the extracted combinations as explanatory variables. In this manner, a machine learning model may be created to have higher accuracy and a higher explanatory property as compared with a case of directly using each descriptor.

The causal relationship generation unit 50 is a processing unit that generates a causal relationship between explanatory variables used to train the machine learning model or a causal relationship between an explanatory variable (including combination of descriptors) and an objective variable. Specifically, the causal relationship generation unit 50 exhaustively checks combinations of features of the machine learning model using a technique of analyzing which factor is a cause and which factor is a result by analyzing mutual influence when two factors mutually change, and extracts a causal relationship for each condition group.

More specifically, the causal relationship generation unit 50 extracts a degree of influence or the like of each combination of descriptors exerted on each condition set as the objective variable of the machine learning model, thereby generating a causal relationship between the descriptor and the objective variable. For example, the causal relationship generation unit 50 sets a "combination that largely affects the objective variable" among the individual combinations extracted by the combination extraction unit 42 as a grouping rule of original data. Then, by analyzing a causal relationship in a specific group, the causal relationship generation unit 50 individually extracts a causal relationship in which multiple causal relationships are mixed and offset each other to be invisible when viewed as a whole.

Describing with reference to FIG. 7, the causal relationship generation unit 50 generates, as a first causal relationship, "influence of kink_1 = 1 and vac_2 = 1 exerted on a reduction of reaction energy of the catalyst is 10.22" or the like. The causal relationship generation unit 50 generates, as a second causal relationship, "influence of the element_1 = 44 (atomic number 4: Ru) and step_1 = 1 and island_1 = 1 exerted on a reduction of reaction energy of the catalyst is 8.74" or the like.

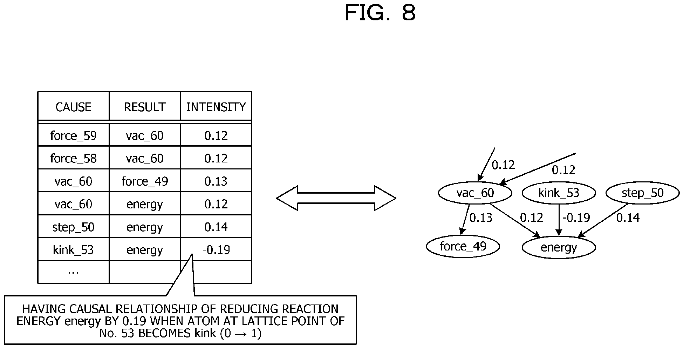

Here, the causal relationship generated by the causal relationship generation unit 50 is represented by the causal relationship between the individual features described above. FIG. 8 is a diagram for explaining a causal relationship. As illustrated in FIG. 8, for example, the causal relationship generation unit 50 analyzes the causal relationship based on a correlation narrowed down by machine learning using a method such as "DirectLiNGAM" or the like, and generates a result represented by the causal relationship between the individual features. That is, the causal relationship generation unit 50 analyzes how much influence is exerted on the value of the "result" when the "cause" increases by 1. In the example of FIG. 8, the causal relationship generation unit 50 generates a causal relationship "when an atom at the lattice point of No. 53 becomes kink (kink_53 = 0 changes to kink_53 = 1), reaction energy is reduced by 0.19". Furthermore, all causal relationships not linked to the energy in FIG. 8 are causal relationships between explanatory variables.

Furthermore, the causal relationship generation unit 50 may output a schematic diagram of the physical property structure of the catalyst, and in the schematic diagram, atoms or lattice points having a causal relationship of equal to or higher than a predetermined value may be highlighted according to the content of the causal relationship. For example, the causal relationship generation unit 50 maps the causal relationships of the catalyst structure generated in analysis of chemical catalyst characteristics on three-dimensional catalyst data, and highlights atoms and lattice points having a particularly strong relationship of equal to or greater than a threshold using a color, a shape, and the like according to the content of the causal relationship.

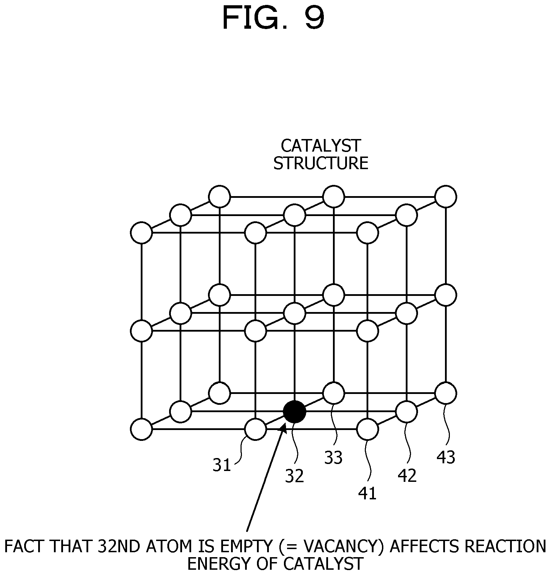

FIG. 9 is a diagram for explaining an exemplary output of the causal relationship. As illustrated in FIG. 9, when "the fact that the 32nd atom is empty affects the reaction energy of the catalyst", the causal relationship generation unit 50 highlights the 32nd atom position of the catalyst structure. As another example, when "the fact that the lattice point 1 has a kink structure and the lattice point 2 has no atom to have a vacancy structure is a cause of reduction in the reaction energy of the catalyst", the causal relationship generation unit 50 highlights the lattice point 1 in red representing a kink causal effect, highlights the lattice point 2 in black representing a vacancy causal effect, and displays other lattice points in white.

As described above, the information processing apparatus 10 according to the first embodiment may estimate and illustrate, using a machine learning model, the influence of the descriptors including the structural characteristic data and combinations thereof exerted on the objective variable. Moreover, the information processing apparatus 10 may estimate, using this machine learning model, the objective variable in a similar manner to normal machine learning.

The prediction processing unit 60 is a processing unit that includes a data generation unit 61 and a prediction unit 62 and performs prediction processing on prediction target data using a machine learning model. Specifically, the prediction processing unit 60 specifies the applicable causal relationship among the causal relationships generated by the causal relationship generation unit 50 based on the prediction target data, thereby specifying, for example, a descriptor having large reaction energy of the catalyst with respect to the prediction target data, and the like.

The data generation unit 61 is a processing unit that generates structural characteristic data from the prediction target data by a method similar to that of the data extraction unit 41. Furthermore, the data generation unit 61 may generate atomic arrangement data from the prediction target data. The data generation unit 61 stores each piece of the generated data in the storage unit 12, and outputs the data to the prediction unit 62.

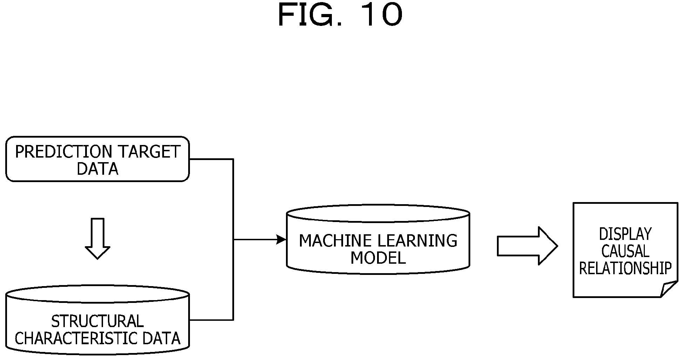

The prediction unit 62 is a processing unit that performs the prediction processing on the prediction target data using a machine learning model. FIG. 10 is a diagram for explaining exemplary prediction using a causal relationship. As illustrated in FIG. 10, for example, the prediction unit 62 generates a plurality of combinations of descriptors from the structural characteristic data and the atomic arrangement data generated from the prediction target data using, for example, the technique of Non-Patent Document 1. Then, the prediction unit 62 inputs the plurality of generated combinations of descriptors to the machine learning model generated by the machine learning unit 40 to obtain a prediction result. As a result, the prediction unit 62 is enabled to obtain the prediction result and to calculate a prediction value of the objective variable.

Furthermore, the prediction unit 62 refers to generated causal relationships generated at the time of training the machine learning model, and specifies a causal relationship corresponding to the combination of descriptors generated from the prediction target data. Then, the prediction unit 62 outputs the specified causal relationship to a display or the like as a prediction result, and transmits it to the administrator terminal. For example, when the combination of "kink_1 = 1" and "vac_2 = 1" is included in the combination of descriptors generated from the prediction target data, the prediction unit 62 determines that it corresponds to the first causal relationship, and predicts that "the descriptor that largely affects the reduction of the reaction energy of the catalyst is included".

### (Process Flow)

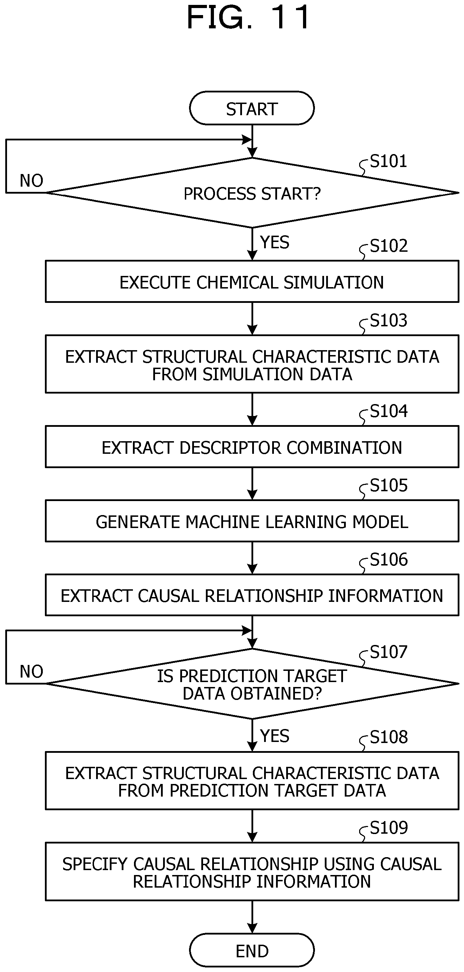

FIG. 11 is a flowchart illustrating a process flow. While an exemplary case of executing the prediction processing after the machine learning processing will be described here, they may be implemented by separate flows.

As illustrated in FIG. 11, when the process starts (Yes in S101), the machine learning unit 40 of the information processing apparatus 10 executes chemical simulation to generate simulation data (S102).

Subsequently, the machine learning unit 40 extracts structural characteristic data from the simulation data (S103). Then, the machine learning unit 40 generates a combination of descriptors from the simulation data and the structural characteristic data (S104).

Thereafter, the machine learning unit 40 executes machine learning using a specified objective variable and the descriptors the combinations thereof as explanatory variables to generate important combinations of descriptors and a machine learning model using the combinations (S105), and generates causal relationship information using the important combinations of descriptors (S106).

Thereafter, upon acquisition of the prediction target data (Yes in S107), the prediction processing unit 60 extracts the structural characteristic data from the prediction target data (S108). Then, at the time of predicting the objective variable from the prediction target data using the machine learning model, the prediction processing unit 60 specifies the corresponding causal relationship using the causal relationship information (S109).

### (Effects)

As described above, the information processing apparatus 10 may automatically extract a feature from the atomic-level simulation data at high speed by extracting only the feature for each atom as a commonly used high-order feature, for example, not the characteristics of the structure including a plurality of atoms, a temperature or pressure given to the cluster of atoms, and the like from the atomic-level simulation data.

The information processing apparatus 10 may compile the feature for each atom by machine learning with a group that largely affects the reaction energy as an output of the atomic-level simulation, which is a group related to the feature for each atom, as a condition, and then apply a causal discovery. As a result, the information processing apparatus 10 may also find a group of features for each atom that has been overlooked by a person, as compared with a case where a person performs a causal discovery by giving a high-order feature, based on the characteristics of machine learning (e.g., wide learning) capable of exhaustively examining all combinations of conditions.

The information processing apparatus 10 may achieve a more efficient catalyst search at low cost in a short time by being used to check a catalyst reaction process and to determine priority of a range in which a catalyst candidate needs to be searched for. In particular, the information processing apparatus 10 may reduce feature design and search axis selection, which are processes that need an advanced assessment by an expert at an initial stage of a search plan, and may search for a promising catalyst composition at high speed. Furthermore, the information processing apparatus 10 may find a causal effect in the reaction more accurately.

The information processing apparatus 10 may reduce the catalyst search range and largely reduce the time and effort for obtaining a result by estimating characteristics that largely affect the catalyst performance to narrow down the search axes (variables and targets to be tested by changing parameters) in the catalyst search to those estimated to exert a "large influence". In other words, the information processing apparatus 10 may make n of Xⁿ of the search range smaller.

The information processing apparatus 10 may narrow down the search range, which is enormous and is not realistic to be handled manually, and which may not be narrowed down by common AI. The information processing apparatus 10 may also provide such a narrowed result (causal relationship) to another AI (machine learning model).

### [Second Embodiment]

In the meantime, while the embodiment of the present invention has been described above, the present invention may be implemented in a variety of different modes in addition to the embodiment described above.

### (Numerical Values, etc.)

The items of the simulation data, the descriptors, the combinations of the descriptors, the items of the structural characteristic data, the causal relationships, and the like used in the embodiment described above are merely examples, and may be changed optionally. In addition, the process flow described in each flowchart may be appropriately modified unless otherwise contradicted.

### (Input of Descriptor)

For example, while the exemplary case where the information processing apparatus 10 extracts a descriptor by chemical simulation or extraction of a surface structure has been described in the embodiment described above, it is not limited to this. For example, the information processing apparatus 10 may receive a descriptor that may not be automatically extracted from an experimenter or an evaluator, and may use it as an explanatory variable.

Furthermore, when a specific descriptor, such as a user-specified descriptor, a descriptor to be evaluated, or the like is an evaluation target, the information processing apparatus 10 may generate a causal relationship using a combination including the specific descriptor. In this case, the information processing apparatus 10 may generate a result desired by the user at a speed higher than that in the case where causal relationships are generated for all descriptors.

### (System)

Pieces of information including the processing procedure, control procedure, specific names, various types of data and parameters described above or illustrated in the drawings may be altered in any way unless otherwise noted.

Furthermore, each component of each device illustrated in the drawings is functionally conceptual, and is not necessarily physically configured as illustrated in the drawings. In other words, specific forms of distribution and integration of individual devices are not limited to the forms illustrated in the drawings. That is, all or a part thereof may be configured by being functionally or physically distributed or integrated in any units depending on various loads, use situations, or the like. For example, the simulation execution unit 30, the machine learning unit 40, the causal relationship generation unit 50, and the prediction processing unit 60 may be implemented by separate computers (housings).

Moreover, all or any part of the individual processing functions performed in the individual devices may be implemented by a central processing unit (CPU) and a program analyzed and executed by the CPU, or may be implemented as hardware by wired logic.

### (Hardware)

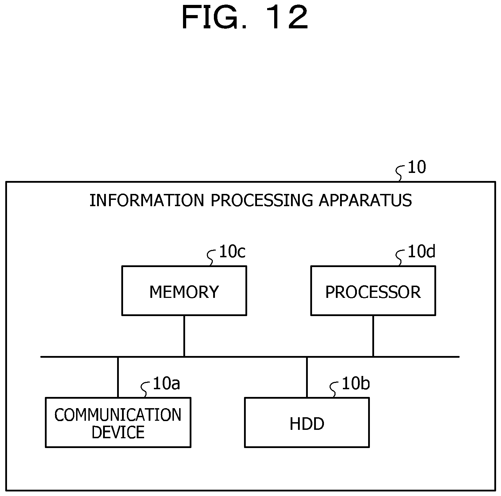

FIG. 12 is a diagram for explaining an exemplary hardware configuration. As illustrated in FIG. 12, the information processing apparatus 10 includes a communication device 10a, a hard disk drive (HDD) 10b, a memory 10c, and a processor 10d. Furthermore, the individual units illustrated in FIG. 12 are coupled to each other by a bus or the like.

The communication device 10a is a network interface card or the like, and communicates with another device. The HDD 10b stores programs and DBs for operating the functions illustrated in FIG. 2.

The processor 10d reads a program that executes processing similar to that of each processing unit illustrated in FIG. 2 from the HDD 10b or the like, and loads it into the memory 10c, thereby operating a process for executing each function described with reference to FIG. 2 and the like. For example, this process executes a function similar to that of each processing unit included in the information processing apparatus 10. Specifically, the processor 10d reads, from the HDD 10b or the like, programs having functions similar to those of the simulation execution unit 30, the machine learning unit 40, the causal relationship generation unit 50, the prediction processing unit 60, and the like. Then, the processor 10d executes a process of performing processing similar to that of the simulation execution unit 30, the machine learning unit 40, the causal relationship generation unit 50, the prediction processing unit 60, and the like.

In this manner, the information processing apparatus 10 operates as an information processing apparatus that executes an information processing method by reading and executing a program. In addition, the information processing apparatus 10 may also implement functions similar to those in the embodiments described above by reading the above-mentioned program from a recording medium with a medium reading device and executing the above-mentioned read program. Note that the program mentioned in other embodiments is not limited to being executed by the information processing apparatus 10. For example, the embodiments described above may be similarly applied also to a case where another computer or server executes the program or a case where these computer and server cooperatively execute the program.

This program may be distributed via a network such as the Internet. In addition, this program may be recorded in a computer-readable recording medium such as a hard disk, a flexible disk (FD), a compact disc read only memory (CD-ROM), a magneto-optical disk (MO), a digital versatile disc (DVD), or the like, and may be executed by being read from the recording medium by a computer.

### REFERENCE SIGNS LIST

- 10: Information processing apparatus

- 11: Communication unit
- 12: Storage unit
- 13: Simulation data DB
- 14: Structural characteristic data DB
- 15: Prediction target data DB
- 20: Control unit
- 30: Simulation execution unit
- 40: Machine learning unit
- 41: Data extraction unit
- 42: Combination extraction unit
- 50: Causal relationship generation unit
- 60: Prediction processing unit
- 61: Data generation unit
- 62: Prediction unit

## Claims

1. A machine learning program for causing a computer to execute a process comprising:
extracting a feature related to a surface structure of a substance based on an atomic arrangement of the substance; and
training a machine learning model that predicts information regarding a chemical reaction that occurs in a substance that corresponds to an input explanatory variable using training data that includes, as an explanatory variable, atomic arrangement information regarding the atomic arrangement of the substance and the extracted feature and includes, as an objective variable, information regarding the chemical reaction that occurs in the substance.

2. The machine learning program according to claim 1, the program causing the computer to execute the process further comprising:
extracting a causal relationship between each of the features set in the explanatory variable and a degree of influence exerted on the objective variable when the training of the machine learning model is complete.

3. The machine learning program according to claim 2, wherein
the extracting extracts the feature related to the surface structure of the substance according to the atomic arrangement information obtained by chemical simulation regarding a catalyst and a condition definition of a surface characteristic of the substance, and
the training includes:
generating, from the atomic arrangement information and the feature, a combination of descriptors that indicates a feature that represents a chemical characteristic of the catalyst; and
generating the machine learning model using the training data that includes the combination of the descriptors as the explanatory variable and the information regarding the chemical reaction as the objective variable.

4. The machine learning program according to claim 3, wherein
the extracting extracts, as the feature, a characteristic regarding a three-dimensional structure of the catalyst according to the atomic arrangement information obtained by the chemical simulation regarding the catalyst and the condition definition of the surface characteristic of the substance.

5. The machine learning program according to claim 3, wherein
the extracting extracts, for each of the descriptors, a causal relationship between the descriptor and the degree of influence exerted on the objective variable.

6. The machine learning program according to claim 3, the program causing the computer to execute the process further comprising:
outputting a schematic diagram of a physical property structure of the catalyst; and
highlighting, according to content of the causal relationship, an atom or a lattice point that has the causal relationship of equal to or higher than a predetermined value in the schematic diagram.

7. The machine learning program according to claim 3, the program causing the computer to execute the process further comprising:
extracting the feature from prediction target data regarding the catalyst to be predicted according to the atomic arrangement information and the condition definition of the surface characteristic of the substance; and
inputting the feature and the atomic arrangement information generated from the prediction target data to the machine learning model, and predicting the chemical reaction in analysis of the catalyst regarding the catalyst to be predicted.

8. A machine learning method implemented by a computer, the machine learning method comprising:
extracting a feature related to a surface structure of a substance based on an atomic arrangement of the substance; and
training a machine learning model that predicts information regarding a chemical reaction that occurs in a substance that corresponds to an input explanatory variable using training data that includes, as an explanatory variable, atomic arrangement information regarding the atomic arrangement of the substance and the extracted feature and includes, as an objective variable, information regarding the chemical reaction that occurs in the substance.

9. An information processing apparatus comprising:
a control unit configured to:
extract a feature related to a surface structure of a substance based on an atomic arrangement of the substance; and
train a machine learning model that predicts information regarding a chemical reaction that occurs in a substance that corresponds to an input explanatory variable using training data that includes, as an explanatory variable, atomic arrangement information regarding the atomic arrangement of the substance and the extracted feature and includes, as an objective variable, information regarding the chemical reaction that occurs in the substance.
